# EUROPEAN PATENT APPLICATION

(11) **EP 2 383 017 A1**
(43) Date of publication of application: **02.11.2011**
(21) Application number: 10161245.5
(22) Date of filing: 28.04.2010
(51) Int. Cl.: A61N 5/06

(54) **Phototherapy device**

(71) Applicant: Koninklijke Philips Electronics N.V., 5621 BA Eindhoven (NL)
(72) Inventor: Wagenaar Cacciola, Giovanna, 5656 AE Eindhoven (NL)
(74) Representative: Damen, Daniel Martijn

(57) **Abstract**

A phototherapy patch (3) is provided for relieving nociceptive pain of a body part by illumination of at least part of the body part. The patch is formed for conforming to at least part of the body part. The patch comprises a first light source for emitting light (9). The patch is arranged for illuminating at least a portion of the body part with light from the first light source. The first light source is configured to emit light with a wavelength in the range of 430 nm to 475 nm.

## Description

### FIELD OF THE INVENTION

The present disclosure relates to devices for pain relief, in particular for pain relief by phototherapy.

### BACKGROUND OF THE INVENTION

People can suffer from two types of physical somatogenic pain, or a combination thereof: neuropathic pain and nociceptive pain. Neuropathic pain is a consequence of damage to and/or malfuction of the nervous system. Nociceptive pain is a consequence of triggering particular nerves, the nociceptors, of a normally functioning central or peripheric nervous system.

Several forms of nociceptive pain, in particular muscular pain and joint pain are known to be relieved by applying heat to the painful location, e.g. via a hot bath or shower, a heat pad such as a chemical or physical heat pad and/or via illumination with infrared light (IR light).

Heat may improve blood circulation and warm muscles. As the muscles are soothed by the heat, they automatically loosen up and relax. At the same time, improved blood circulation helps the irradiated body part get rid of impurities and quickly sends oxygen-rich blood to stressed or aching muscles, bringing effective pain relief. In joints, heat may assist restoring the right viscosity of the synovial fluid to improve its lubricating and nourishing properties for the joint, thus reducing joint pain. Heat may further stimulate production of endorphins.

US 2004/166146 discloses a phototherapy bandage capable of providing radiation to a localized area of a patient for accelerating wound healing and pain relief, photodynamic therapy, and for aesthetic applications. The phototherapy bandage may include a flexible light source that is continuous across the bandage for providing a selected light, such as a visible light, a near-infrared light, or other light, having substantially similar intensity across the bandage. The bandage may also be flexible and capable of being attached to a patient without interfering with the patient's daily routine. The phototherapy bandage may easily conform to the curves of a patient and may come in a variety of exterior shapes and sizes.

In the present disclosure phototherapy patches are disclosed which provide improvements in relieving pain.

### SUMMARY OF THE INVENTION

A phototherapy patch is provided for relieving nociceptive pain of a body part by illumination of at least part of the body part. The patch is formed for conforming to at least part of the body part. The patch comprises a first light source for emitting light. The patch is arranged for illuminating at least a portion of the body part with light from the first light source. The first light source is configured to emit light with a wavelength in the range of 430 nm to 475 nm.

The patch being formed for conforming to at least part of the body part, e.g. by being at least partly flexible, facilitates donning and wearing the patch without hindrance to a subject or a patient and his(/her) movements. The patch may be flexible, textile-like and/or plaster-like and may be provided with means for attaching the patch to a body part, e.g. one or more straps, sticky portions and/or cuffs, preferably allowing fixation of the position of the patch with respect to the portion of the body part to be illuminated.

Light with a wavelength in the range of 430 nm to 475 nm, violet to blue, is predominantly absorbed by the epidermis and dermis of a human. Absorption and reflection of light at such wavelengths depends on the skin color of the subject; fair skin reflects more than dark skin. However, it has been found that light at such wavelengths is perceived quite well by human skin heat sensors, better than IR light, and provides a sensation of warmth which may be very comfortable to the subject. Further, it has been found that the optical energy deposited in the skin is transformed to heat which will warm up the illuminated body part with the above-described curative effects. This in depth thermal effect of blue-violet light has been found to appear slower than what can be achieved by IR illumination but, once in a steady state, to be substantially equal to the thermal effect of IR irradiation. It has further been found that light at such wavelengths promotes the formation of nitric oxide in the tissue. Nitric oxide tends to incite vasodilation, thus improving blood flow, and to reduce inflammation.

The wavelength range of the first light source may advantageously be 430-470 nm, such as 440-460 nm. It has been found that the reflection of the skin increases for increasing wavelengths in the visible range. Thus the effectivity of optical energy applied with longer blue light wavelengths is reduced. For decreasing wavelengths the absorption of the optical energy increases, but possibly undesired side effects may increase as well e.g. skin tanning and/or damaging. Such behaviour is substantially equal for both fair and dark skin types, be it at different absolute reflectivity. A first light source which is configured to emit light with a predominant wavelength of approx. 450 nm, e.g. having a spectral bandwidth of about 1 to 5 nm centered around 450 nm, is considered suitable.

Efficient light sources at such wavelenghts are Light Emitting Diodes (LEDs). Thus, in an efficient patch the first light source may comprise at least one LED. A patch comprising a plurality of LEDs may facilitate uniform illumination of a significantly larger surface area than when comprising a single LED. LEDs may generate little heat per emitted optical energy compared to other types of light sources. Any heat that is, after all, generated by a patch comprising LEDs may be used to benefit treatment, as in a warmth pad. Presently, relatively powerfull LEDs with an emission peak at approx. 450 nm and a full-width-at-half-maximum spectral bandwidth of approx. 20 nm are readily available and have proven particularly suitable.

To further increase treatment efficiency, in particular when commencing a treatment session, the patch may comprise a second light source which is arranged for illuminating the body part with near-infrared light (near-IR) having a wavelength in the range of approx. 800-2500 nm, in particular 800-1500 nm (IR-A), and with the patch being arranged for, when in use, illuminating at least a portion of the body part with light from the second light source. Such near IR light is reflected by human skin more than violet or blue light, uptil ~1200nm, but, once transmitted, it is absorbed predominantly in the dermis, and the subcutis. Thus, deeper treatment may be provided more directly, but the radiation is felt less, so that IR light provides a reduced sensation of warmth in comparison to blue light. The penetration depth of near-IR light into human tissue through the skin has a global maximum absorption for near-IR light at approx. 1500 nm, and a local maximum at approx. 2200 nm, with a local minimum in between at approx. 1950 nm.

Further, most consumers know that IR radiation is thermal radiation. Acceptance of (the use of) a phototherapy patch for pain relief may thus be improved when the patch comprises an IR light source.

IR-A light may efficiently be provided with one or more LEDs. Powerfull LEDs in the range 800-1500 nm are commercially available at moderate cost.

LEDs for blue and/or IR-A light can be rapidly switched and may be well controllable with respect to intensity. LEDs may have emission bandwidths of several nanometers, the emission spectrum also depending on the temperature of the LED and/or the power with which it is driven. This facilitates control over (the light emitted by) the patch. A patch comprising LEDs may comprise (combinations of) one or more separately packaged LEDs, encapsulated LEDS for one or more wavelengts, flat surface-emitting LEDs, textiles comprising LEDs such as Lumalive® technology, etc.

It has been found that by illuminating skin with sufficiently intense radiation a subject may clearly feel the illuminated spot. By modifying the intensity distribution with which a subject is illuminated in time and/or space, a massaging effect may be imparted to the illuminated body part. This is particularly the case when the modification is slower than adaption processes in the tissue, for faster modification the sensation may appear continuous. Because the blue is perceived by the skin heat sensors more easily than IR-A, the adaptation processes, e.g. the modulation depth (difference between period of or location with relatively high intensity and period of or location with relatively low intensity) and modification velocity, may differ for both wavelenghts. Thus, it is conceivable to use near-IR light for an in-depth treatment at a base-level and use blue light in addition to achieve a desired illumination level, and to modify the blue light to provide a massage at the same time. It has been found that such effect may be achieved with sufficiently strong light at the cited wavelengths but it is most easily provided and controlled with LEDs.

Alternatively or in addition, the second light source may comprise a photoluminescent material, e.g. nanocrystals and/or a phosphor, in particular a fluorescent phosphor in the form of a remote phosphor, for transforming incident light of one wavelength and emitting light of another wavelength. Photoluminescent materials are available with various absorption and emission characteristics facilitating manufacture of a patch with desired optical properties. Phosphors may further have long operational lifetimes, comparable to LEDs.

In an efficient phototherapy patch, the first light source may be arranged for illuminating and exciting the photoluminescent material of a photoluminescent second light source. Thus, a single light source need be powered to provide treatment light with plural desired wavelengths. It is noted that the longer the wavelenght generated by the photoluminescent second light, the more efficient the generation of such second light may be by excitation of the photoluminescent material with first light of a shorter wavelength, such as supplied by a first light source emitting blue-light. A patch emitting blue light at 450 nm and near-IR light at approx. 2200 nm may therefore be more energy-efficient than a patch emitting blue light at 450 nm and near-IR light at approx. 1500 nm.

Photoluminescent material, in particular a dusty or fine-grain particulate material embedded in, coated, glued or painted on a substrate, etc., may be used for providing a desired intensity distribution. The photoluminescent material may also be patterned i.e. applied in a predetermined pattern on and/or in a substrate, to provide a desired predetermined intensity distribution. The intensity distribution may be defined for both the first and second light sources. E.g. a random or regular pattern may comprise denser portions for relatively intense emission of IR light interspersed with less dense or open portions for (partial) transmission of blue light from the first light source.

The patch may comprise a light guide for receiving and redistributing at least a portion of the light from the first and/or second light source, e.g. for redistributing light from a first intensity distribution to a second intensity distribution which differ from each other in at least one of an intensity and a direction of propagation. E.g., the patch may be substantially flat, extending substantially in a plane in bandage-like or plaster-like form. The light guide may then also be substantially flat and redistribute the light from the first and/or second light source from substantially parallel to or in the plane of extension of the patch to a direction substantially normal to the patch for illuminating a body part. This allows arranging the respective light source aside from an optically active portion of the patch and facilitates manufacturing a patch whith limited user interference e.g. being inconspicuous under clothing and/or preventing hindering motion. The light guide may act as a diffuser or rather a concentrator between at least the first light source and an optically active portion of the patch.

The light guide may comprise at least one of a flexible sheet-like material and a fiberoptic material. Such flexible sheet-like material may comprise a flexible, at least partially translucent, slab or foil, e.g. one or more ribbon-like or sheet-like portions. A fiberoptic material may comprise one or more optical fibers, splitters, and/or fiberoptic mats, possibly woven, knitted and/or otherwise worked to form a textile. Combinations are also conceivable. The flexibility of the patch should advantageously be such, that the patch may closely follow the shape of the body part. Different patches may be provided for different body parts, which may include differences in the optical power and/or wavelength delivered by the first and/or second light sources.

In an advantageous embodiment, the light guide and the second light source may be integrated, such as by inserting and/or mixing one or more photoluminescent materials in the light guide material, or coating at least part of a surface of the light guide with a photoluminescent material.

For controlling operation of at least the first light source of the patch, e.g. with respect to output power or intensity distribution in case the first light source comprises plural lights such as plural LEDs, the patch may comprise a controller. The controller may be configured to operate the first and/or second light sources so as to modify at least part of the spatial illumination intensity distribution of the patch in at least one of a spatially-varying and a temporally-varying pattern, to provide a dynamic illumination and possibly a massaging effect. A varying pattern may spread the feeling of heat due to the illumination intensity and thus prevent an occasional local unpleasant feeling or even inadvertent pain, whereas overall a desired high optical energy may be deposited in the tissue to be treated. Thus, user comfort is increased.

To provide a predetermined illumination, the controller may be programmable. A program may comprise a predetermined modification function for operation and/or adjustment of the first and/or second light source, e.g. for massaging, in dependence of treatment parameters and/or input user data. Such program may be used for customisation and/or personalisation of the patch for use with different afflictions and/or subjects.

The patch may comprise a user interface for receiving user input. The controller may be configured for controlling the first and/or second light source as a function of the user input. This further increases user friendliness and versatility of the patch. E.g. with such patch a user, advantageously the subject him-/herself to be treated, may modify the intensity distribution, e.g. to increase user comfort and/or safety.

The user interface may comprise any suitable input device. Useful input devices comprise a wired or wireless remote controller. The patch may comprise an indicator with a (schematic) indication of one or more operating parameters of the patch.

The controller may be programmable with a program, which may define a variation sequence. A program may be provided by hand via the user interface and/or it may be obtained from a data storage medium e.g. a CD, DVD, memory card, USB-stick, internal memory of the patch, or remote sources via the Internet etc.

The patch may suitably comprise a memory for storing one or more settings, functions, programs, etc. The patch may comprise an output for outputting data stored in the memory, e.g. on a data storage medium etc. discussed above for a program. This allows portability of such data to another patch and/or sharing such data with a therapist.

These and other aspects will be apparent from and be elucidated with reference to the embodiments described hereinafter.

### BRIEF DESCRIPTION OF THE DRAWINGS

In the drawings:
Fig. 1 indicates a person wearing a phototherapy patch;
Figs. 2-5 show different embodiments of a phototherapy patch;
Fig. 6 shows a light guide for use in a phototherapy patch;
Fig. 7 shows yet another embodiment of a phototherapy patch.

### DETAILED DESCRIPTION OF EMBODIMENTS

It is noted that in the drawings, like features may be identified with like reference signs. It is further noted that the drawings are schematic, not necessarily to scale and that details that are not required for understanding the present invention may have been omitted. The terms "upward", "downward", "below", "above", and the like relate to the embodiments as oriented in the drawings. Further, elements that are at least substantially identical or that perform an at least substantially identical function are denoted by the same numeral, where useful individualised by an alphabetic suffix.

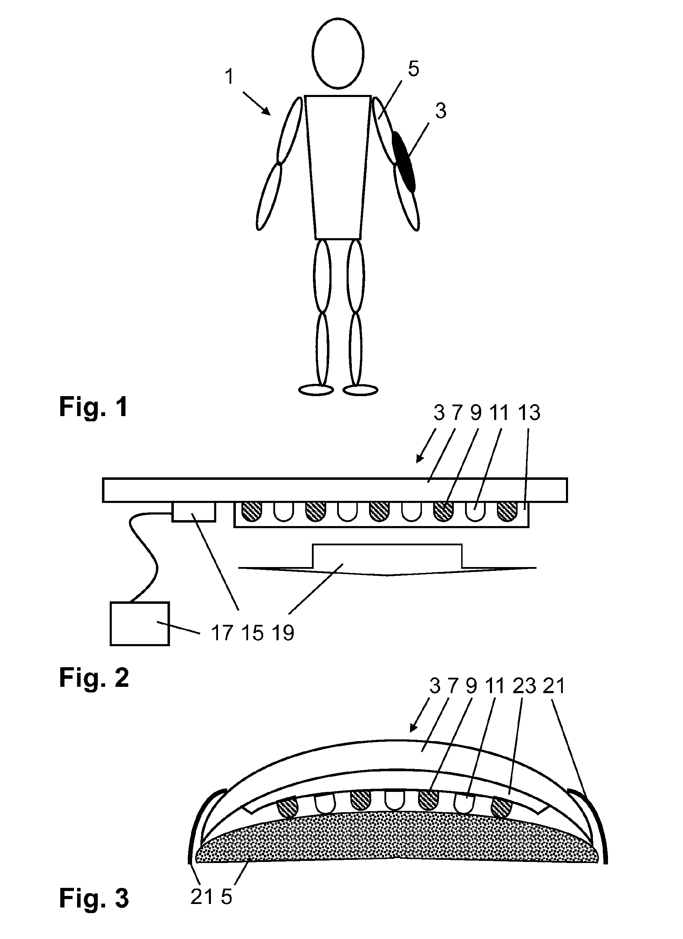

Fig. 1 shows a person 1 carrying a phototherapy patch 3 on a body part 5 of the person 1 (here on an elbow). A cross-section of the patch 3 is shown in Fig. 2. The patch 3 comprises a flexible carrier 7, to which a plurality of light sources 9, 11 are attached. The first light sources 9 are configured to emit light with a wavelength in the range of 430 nm to 475 nm. The second light sources 11 are configured to emit light with a wavelength in the range of 800 nm to 1500 nm. In the shown embodiment the light sources 9, 11 comprise encapsulated LEDs which are partially embedded in an optional flexible padding layer 13. However, different light sources, e.g. non-encapsulated and/or surface-emitting LEDs may be used also or alternatively, which may result in a thinner patch 1. Preferably, the first and/or second light sources 9, 11 are covered with a transparent cover and/or one or more lenses which allow easy cleaning. The carrier 7 comprises electrical conductors (not shown), and/or a flexible PCB via which the light sources 9, 11 are connected to an optional controller 15 and to a power source 17, here in the form of a (possibly rechargeable) battery pack 17 but any other suitable power source may be employed. The light sources 9, 11 are arranged to emit light in a direction substantially normal to the patch 3 to provide an optically active portion of the patch 3, which is indicated with a wide arrow 19. Each light source 9, 11 generates a somewhat divergent light cone such that a substantially homogeneous intensity distribution is provided at a distance from the light sources 9, 11. The exact arrangement of the light sources 9, 11 and their respective light cones determines the resulting intensity distribution.

The controller 15 and/or the power source 17 may be arranged elswhere with respect to (the carrier 7 of) the patch 3, e.g. on an exterior side opposite the light sources 9, 11 and/or partially comprised in the carrier 7.

Fig. 3 indicates, in cross section, another patch 3 conforming to a body part 5 to which the patch 3 is attached by means of a strap 21 looped around the body part 5 and closed in any suitable manner, such as with a clasp, a buckle, a button, a knot, hook-and-loop-type fastener (e.g. Velcro®), etc. Here, the the light sources 9, 11 are arranged on an optional flexible intermediate layer 23 attached to the flexible carrier layer 7. The carrier layer 7 here has a decreasing thickness towards its edges to reduce possible dragging to clothes and to improve user comfort.

In the embodiments of Figs. 2 and 3 the first light sources 9 and/or the second light sources 11 may be operated independently, e.g. to flash consecutively as running lights to provide a spatially dynamic massaging effect. Contemporaneous flashing may provide a massaging effect in a stationary position. Both effects may be combined. "Flashing" as used here, should be interpreted as oscillating regularly or irregularly the emitted intensity of the light source(s) 9, 11 concerned between intensity levels separated by a modulation depth of sufficient amount to be percieved, in particular between maximum and minimum intensity levels wherein a minimum intensity level is less than approx. one quarter the intensity of the directly preceding maximum intensity level. Flashing may include fully switching off a light source to provide a large modulation depth. Due to the difference in perception for blue and IR light, the blue light may be used for providing a clearly discernible massaging effect whereas the IR light is continuously operated. Thus, pain treatment and user comfort can be optimised together.

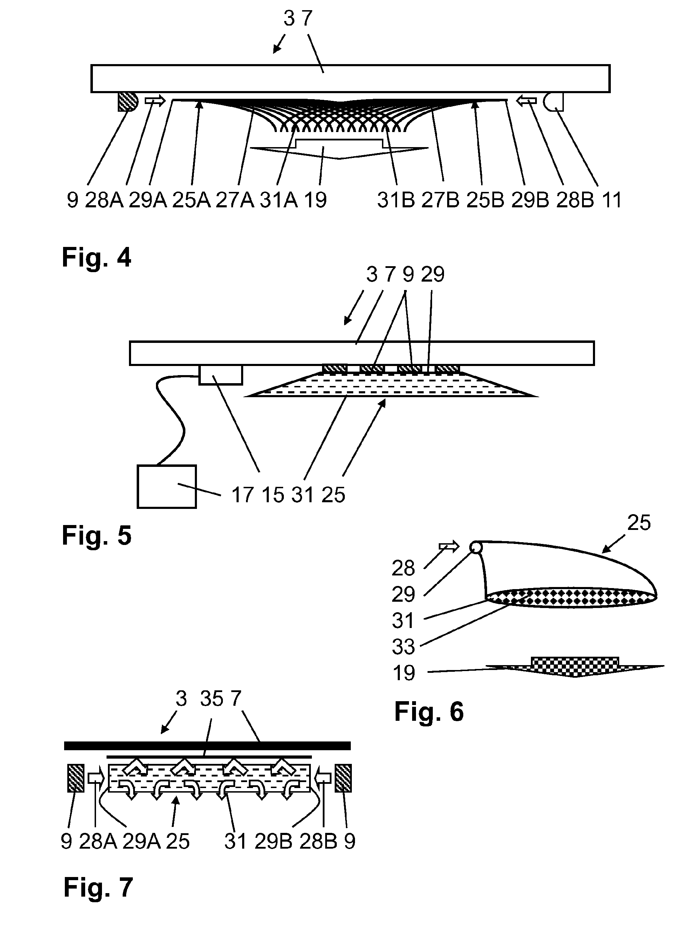

Fig. 4 indicates an embodiment of a patch comprising light guides 25A, 25B in the form of bundles of fiberoptic material attached to a flexible carrier 7. In the shown patch, one first light source 9 illuminates one end 29A of a bundle of optical fibers 27A (indicated with arrow 28A). At the end 29A, (the ends of) the fibers 27A are arranged close together. The light received in the fibers 27A is guided through the fibers 27A and re-emitted from an opposite end 31A of the fiber bundle (see arrow 19). At the opposite end 31A (the ends of) the fibers 27A are spread out over an area which is different and substantially larger than at the end 29A, to provide a relatively large effective optical area for the patch 3. In the embodiment of Fig. 4, a second light source 11 is arranged at an opposite side of the patch 3 and is arranged for illuminating (arrow 28B) another light guide 25B comprising a bundle of optical fibres 27B extending between a first end 29B and a second end 31B in similar fashion the what is described above. The ends 31A and 31B overlap by having the ends of the respective fibers 27A, 27B arranged in interspersed fashion in any desired random or regular pattern.

As will be apparent from Fig. 4, the first and second light sources 9, 11, again shown as encapsulated LEDs for exemplary purposes and for facilitating comparison to Figs. 2 and 3, may be arranged for emitting substantially parallel to the carrier 7 (cf arrows 28A, 28B) whereas the light effectively is emitted normal to (the carrier 7 of) the patch 3 via the light guide 25A, 25B.

Fig. 5 shows a patch 3 comprising surface-emitting LEDs 9 for emitting light with a wavelength of approx. 450 nm. The patch 3 further comprises a tapered light guide 25 having a first end 29 with a relatively small surface area and a second end 31 with a relatively large surface area. The light guide 25 comprises a material mixed with a photoluminescent material which is excitable by the light of the first light sources 9. The first light sources 9 and the light guide 25 are arranged such that the light sources 9 illuminate the first end 29 of the light guide 25. A first portion of the light of the first light sources 9 received in the light guide 25 is emitted redistributed over the surface area of the second end 31. A second portion of the light of the first light sources 9 received in the light guide 25 excites the photoluminescent material, which subsequently fluoresces at one or more wavelengths in a range between 800 and 1500 nm. This light is also emitted distributed over the surface area of the second end 31. Thus, the light guide 25 serves the double purpose of being a redistributing light guide and being a light source for a second wavelength, i.e. a second light source. The blue light and the IR light are substantially homogeneously mixed. The relative intensities of the blue light and the IR light may be determined by selecting the (relative) amount of photoluminescent material in the light guide 25, and possibly its location, during manufacuring of the light guide.

Fig. 6 illustrates a light guide 25 which is a solid, but flexible, object, e.g. a piece of silicone material which is transparent to at least the first light source's wavelength. The light guide 25 of Fig. 6 comprises a first end 29 with a relatively small surface area and a second end 31 with a relatively large surface area. Other shapes of a light guide 25 in between a first end 29 with a relatively small surface area and a second end 31 with a relatively large surface area are conceivable and may be used. On the surface of the second end 31 a coating 33 is applied comprising a fluorescent phosphor which is photoexcitable by the light from the first light source 9 to emit light with a wavelength in the range of 800-1500 nm. The coating 33 is arranged in a chessboard-pattern, leaving portions free from coating material. Thus, in use and when illuminated by a first light source 9 at the first end 29 (see arrow 28), light with a chessboard-pattern of wavelengths is emitted from the second end 31 (see arrow 19). The chess-board pattern may be arranged such that a substantially homogeneous illumination of the body part 5 to be treated is applied. Other patterns, e.g. stripes, dots, combinations, aperiodic, etc. may be used and the size of pattern features, e.g. the size of squares in a chess-board pattern may be selected to provide a desired effect.

Fig. 7 illustrates a patch 3 comprising side-illumination of a flexible light guide 25 by blue light sources 9 via first ends 29A, 29B. The light guide 25 comprises a material mixed with a photoluminescent material which is excitable by the light of the blue light sources 9, emitting near-IR light. The resultant blue and near-IR light is scattered within the light guide 25, possibly being reflected by an optional reflector 35, to be emitted substantially homogeneously from the light guide 25 via second end 31 (see the open white arrows).

In an embodiment comprising fiberoptic material, one or more optical fibers may be optimised for transmission of light, and one or more fibers may be optimised for leaking light, e.g. by roughening the fiber surface and/or by having an asymmetrically shaped core. Thus, a more homogeneous illumination may be provided.

One or more fibers may be doped and/or coated with a photoluminescent material which is photoexcitable by blue light in the wavelength range 430-475 nm to emit light in the wavelength range 800-1500 nm, so as to perform the double function of light guide and second light source.

One or more light sources may be fibre-coupled to one or more fibres of a fiberoptic light guide e.g. pigtailed.

Combinations of an integrated light guide and second light source with individual second light sources configured to emit IR light in the wavelength range 800-1500 nm may be applied to allow modifying the illumination intensity and/or pattern of the IR light independent of modification of the blue light from the first light sources.

A patch may comprise one or more reflectors to improve directing light to a desired location and/or providing a desired illumination distribution.

A patch may comprise plural flexible light guides which may be stacked. Preferably, each light guide (to be) arranged closer to the body part to be illuminated is substantially translucent for light emitted and/or transmitted from a light guide arranged closer to a flexible carrier and/or arranged for being further away from the body part to be illuminated. Different light guides may have different properties.

A patch may be water resitant, allowing wearing in outdoors situation and/or during sports. A patch may be formed for use with an at least partially translucent fluid e.g. a gel, for outcoupling and transmitting light to the portion to be illuminated. One or more portions of the patch may be washable.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. Phototherapy patch (3) for relieving nociceptive pain of a body part (5) by illumination of at least part of the body part
wherein the patch is formed for conforming to at least part of the body part
wherein the patch comprises a first light source (9) for emitting light with a wavelength in the range of 430 nm to 475 nm
wherein the patch is arranged for illuminating at least a portion of the body part with light from the first light source.

2. The phototherapy patch (3) of claim 1, wherein the first light source (9) comprises at least one Light Emitting Diode (9).

3. The phototherapy patch (3) of claim 1 or 2, wherein the patch comprises a second light source (11)
wherein the second light source is arranged for illuminating the body part (5) with near-infrared light having a wavelength in the range of 800 nm to 2500 nm,
wherein the patch is arranged for, when in use, illuminating at least a portion of the body part with light from the second light source.

4. The phototherapy patch (3) of claim 3, wherein the second light source (11) comprises at least one Light Emitting Diode (11).

5. The phototherapy patch of claim 3 or 4, wherein the second light source (11) comprises a photoluminescent material (33), e.g. a phosphor.

6. The phototherapy patch (3) of claim 5, wherein the first light source (9) is configured to illuminate and excite the photoluminescent material of the second light source (11).

7. The phototherapy patch (3) of claim 5 or 6, comprising a patterned photoluminescent material (33).

8. The phototherapy patch (3) of any preceding claim
wherein the patch comprises a light guide (25, 25A, 25B) for receiving and redistributing at least a portion of the light from at least the first light source (9).

9. The phototherapy patch (3) of claim 8, wherein the light guide (25, 25A, 25B) comprises at least one of a flexible sheet-like material and a fiberoptic material.

10. The phototherapy patch (3) of any preceding claim, wherein the patch (3) comprises a controller (15) for controlling operation of at least the first light source (9).

11. Method of relieving nociceptive pain of a body part (5) comprising:
applying a phototherapy patch (3) which is formed for conforming to at least part of the body part to be treated, and which comprises at least a first light source (9),
illuminating of at least part of the body part with light from the first light source (9) having a wavelength in the range of 430 nm to 475 nm.

12. The method of claim 11, the patch (3) further comprising a second light source (11), wherein the method further comprises:
illuminating of at least part of the body part with light from the second light source (9) having a wavelength in the range of 800 nm to 2500 nm.

13. The method of claim 11 or 12, further comprising: operating at least one of the first and second light sources (9; 11) such that the light for illumination of the body part is provided to the body part (5) with an intensity distribution which is varied in at least one of spatially and temporally in a predetermined pattern.

14. A storage medium comprising software code portions for, when executed by a controller of a phototherapy patch according to at least claim 10, operating at least one of the first and second light sources (9; 11), e.g. for performing the method of any one of claims 11-13.
